(19) Europäisches Patentamt
European Patent Office
Office européen des brevets

(11) Publication number: **0 146 842**
**B1**

(12) # EUROPEAN PATENT SPECIFICATION

(45) Date of publication of patent specification: **01.04.87**

(51) Int. Cl.$^4$: **C 07 K 7/36**

(21) Application number: **84114768.9**

(22) Date of filing: **05.12.84**

(54) **Novel calcitonin and collection thereof.**

(30) Priority: **08.12.83 JP 230593/83**

(43) Date of publication of application:
**03.07.85 Bulletin 85/27**

(45) Publication of the grant of the patent:
**01.04.87 Bulletin 87/14**

(84) Designated Contracting States:
**DE FR GB**

(56) References cited:
**US-A-3 926 938**

(73) Proprietor: **MITSUBISHI PETROCHEMICAL CO., LTD.**
**5-2, 2-chome, Marunouchi**
**Chiyoda-ku Tokyo 100 (JP)**

(73) Proprietor: **Mitsubishi Yuka Pharmaceutical Co., Ltd.**
**3-7, Ginza 8-chome Chuo-ku**
**Tokyo104 (JP)**

(72) Inventor: **Matsuo, Hisayuki**
**6653, Oaza Kihara Kiyotake-cho**
**Miyazaki-gun Miyazaki-ken (JP)**
Inventor: **Kangawa, Kenji**
**1520-24, Aza Kushima Oaza Kanoukou**
**Kiyotake-cho Miyazaki-gun Miyazaki-ken (JP)**
Inventor: **Hirose, Sachio c/o Central Research Laboratories**
**Mitsubishi Petroche. Co., Ltd. 1315, Oaza Wakaguri**
**Ami-machi Inashiki-gun Ibaraki-ken (JP)**
Inventor: **Watanabe, Motoo c/o Central Research Laboratories**
**Mitsubishi Petroche. Co., Ltd. 1315, Oaza Wakaguri**
**Ami-machi Inashiki-gun Ibaraki-ken (JP)**

**EP 0 146 842 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Inventor: **Homma, Tamotsu c/o Central Research Laboratories**
**Mitsubishi Petroche. Co., Ltd. 1315, Oaza Wakaguri**
**Ami-machi Inashiki-gun Ibaraki-ken (JP)**
Inventor: **Adachi, Hidenari**
**16-6, Shinmori 5-chome**
**Asahi-ku Osaka-shi Osaka (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

**Description**

BACKGROUND OF THE INVENTION

This invention relates to a nopvel calcitonin and a process for collecting the same, more particularly to a novel calcitonin derived from avian ultimobranchial glands having a physiological activity to lower the calcium concentration in blood and also to a process for collecting the same.

Calcitonin (hereinafter called as "CT") is a peptide hormone participitating in metabolism of calcium, which exists in ultimobranchial glands of avian, fishes, cyclostomata, etc. and in thyroid gland of mammals. It is a single chain polypeptide consisting generally of 32 amino acids, of which the first and the seventh amino acids have an internal disulfide bridge, with the carboxylic end being prolineamide. Up to date, CTs have been extracted and purified from porcine, bovine, sheep, human, rat, salmin and eel, and different peptide structures depending on the animal species have been clarified. In the case of avian, an approximate amino acid composition has been estimated from a crude fraction exhibiting the aforesaid physiological activity [A. Nieto et al., Biochim. Biophy. Acta, *322*, 383 (1973)], but its amino acid composition and amino acid sequence have not yet been clarified.

SUMMARY OF THE INVENTION

In view of the state of the art as mentioned above, the present inventors were interested in the fact that CT has different peptide structures depending on the animal species and also that the calcitonin extracted from ultimobranchial glands exhibits generally high potency, and have made extensive studies with an aim to clarify the structure of a novel CT by extracting and purifying CT from chicken ultimobranchial gland. As a consequence, it has now been found that a novel CT can be isolated by extracting the chicken ultimobranchial gland with an aqueous acidic solution and thereafter applying specific treatments on the extract. Also, the present inventors were successful in determining its structure to accomplish this invention.

More specifically, this invention provides a novel CT represented by the following formula (I) and its salt:

$$\text{H-}\overline{\text{Cys-Ala-Ser-Leu-Ser-Thr-Cys}}\text{-Val-Leu-Gly-Lys-Leu-Ser-Gln-Glu-Leu-His-Lys-Leu-Gln-Thr-Tyr-Pro-Arg-Thr-Asp-Val-Gly-Ala-Gly-Thr-Pro-NH}_2 \quad \text{(I)}$$

wherein Cys represents cysteine, Ala alanine, Ser serine, Leu leucine, Thr threonine, Val valine, Gly glycine, Lys lysine, Gln glutamine, Glu glutamic acid, His histidine, Tyr tyrosine, Pro proline, Arg arginine and Asp aspartic acid,

and also a process for collecting the novel CT represented by the above formula (I) and its salt, which comprises extracting an avian ultimobranchial gland with an aqueous acidic solution and thereafter applying at least one of the following treatments (a) to (d) on the extract:

(a) to precipitate and remove the higher molecular compounds by addition of a hydrophilic organic solvent to the aqueous solution containing the desired product;

(b) to recover basic fractions with the use of a cation exchange resin;

(c) to recover fractions with molecular weights of 3000 to 4000 by gel chromatography; and

(d) to recover fractions having CT-like physiological activity by high performance liquid chromatography (HPLC).

DESCRIPTION OF PREFERRED EMBODIMENTS

First, the novel CT having a physiological activity to lower the calcium concentration in blood according to this invention has the chemical properties as shown below:

(1) Appearance: white powder;

(2) Solubility in solvent: soluble in water, particularly an aqueous acidic solution, but insoluble in chloroform, carbon tetrachloride, benzene, hexane and the like;

(3) Molecular weight: 3,370.7;

(4) Basicity: Salmon CT I $>$ CT of this invention $\geqq$ Eel CT;

(5) Primary structure: the above formula (I);

(6) Immunological properties: CT detectable by use of anti-eel CT serum and anti-salmon CT I serum cross-reacted with the CT of this invention;

(7) Biological activity: 3,000—6,000 MRCU/mg,

(Bioassay procedure):

Bioassay for the hypocalcemic activity was performed with 100 g male Wistar rats fasted for 24 hours. The samples and the standard peptide, dissolved in 0.1 ml of 1% sodium acetate (pH 4.0) containing bovine serum albumin (0.1%), were each injected intravenously. After 1 hour, rats were anesthetized with ethyl ether and bled by puncturing the cardiac with disposable syringe. The serum was obtained by centrifugation and serum calcium was determined by the method of Conerty et al. [Conerty, H.V., et al., Am. J. Clin. Path., *45*, 290 (1966)]. Assay was carried out using five dose (three response per dose). MRC Thyroid Calcitonin Research Standard B, which was gifts from the National Institute for Medical Research Mill Hill,

London, was used as an assay standard. Protein determination of samples were performed by amino acid analysis, and specific biological activity (MRCU/mg) was determined. The amount necessary for lowering $Ca^{2+}$ concentration by 10% is defined as mMRC (Medical Research Council) U;

(8) Duration of pharmacological effect: CT of this invention > Eel CT > Salmon CT I, (Measuring method):

A CT solution corresponding to 20 mMRCU was injected intravenously and the periodical change of $Ca^{2+}$ concentration in serum was measured.

The CT of this invention is a novel polypeptide and has a structure which is entirely different from those of CT's derived from various species of animals known in the art. The biological activity of the CT of this invention is about 5000 MRCU/mg, which is equal to or higher than that of the eel CT or the salmon CT I, and higher by 10 times or more than the human CT, the rat CT, the bovine CT or the porcine CT. Further, as for the duration of pharmacological effect, which is an important element as the peptide pharmaceutical, the CT of this invention is longer than the eel CT or the salmon CT having higher biological activity, thus indicating high potentialities of the CT of this invention as the pharmaceutical with respect to biological activity and pharmacological effect. Thus, the novel CT is a hypocalcemic hormone which plays a very important role in Ca metabolism, and it may be considered to be useful as a pharmaceutical. More specifically, CT is expected to have a wide scope of effects, including therapy of digestive hypercalcemia, bone Paget's disease, osteoporosis, etc. for having potent bone resorption inhibiting action, the effect against digestive ulcer and acute pancreatitis for having an inhibiting action against secreation of gastric acid and pancreatic juice, the effect against chronic articular rheumatism for having antiinflammatory action. Further, examples of clinical application of CT are, in addition to those mentioned above, bone metastasis of malignant tumors, renal osteodystrophy, fracture, scleroderma, etc. Wider scope of applications of CT may be expected in the future as its acting mechanism is clarified [Pharmacia, *19*, 187 (1983)].

Next, the process for collecting the CT of this invention is to be described in detail.

As the starting material to be extracted, ultimobranchial glands of chicken are generally employed. Ultimobranchial glands are recognized of its existence before and after hatching, and it is a spherical organ of about 1 mm in diameter in adult chicken, which is located adjacent to the total carotid below the thyroid gland and parathyroid. The weight of ultimobranchial gland and the CT content therein vary depending on the chicken week-old. When considering availability of chickens in a large amount with homogeneous quality, suitable sizes of ultimobranchial glands as well as easiness in delivery thereof, the chickens to be employed should preferably be of broiler species of 6 to 80 weeks-old.

Extraction of CT from the starting material may be performed as described below.

For example, the starting material is crushed and mixed in about 5 to 10-fold (by weight ratio) of an aqueous acidic solution, preferably a volatile aqueous acidic solution (e.g. an aqueous solution of formic acid or acetic acid) to obtain a suspension of the chicken CT. In this case, the acidic aqueous solution should preferably have pH of 1 to 5. Subsequently, by centrifugation of this suspension, a slightly transparent CT extract is formed at the intermediate layer. Here, the precipitate is primarily constituted of tissue strips of ultimobranchial glands and insolubles, and the uper layer is primarily a mass of white fat. The intermediate layer is collected and provided for subsequent treatment steps.

Purification of the extract obtained as described above can be conducted according to any one of the treatment methods (a) to (d) as mentioned above, but it is preferred to employ a combination of two or more methods.

In the treatment (a), a hydrophilic organic solvent (e.g. lower alochol, acetone, etc.) which does not precipitate the CT of this invention (molecular weight: 3,370.7), in an amount enough to precipitate compounds having higher molecular weight than that of the CT, is added to the above extract to remove the higher molecular compounds.

In the treatment (b), the extract or the treated solution thereof is subjected to cation exchange chromatography for recovery of basic fractions. The chromatography is carried out preferably in a water solvent but it may also be carried out in an aqueous solution in which a hydrophilic organic solvent such as lower alcohols, acetone, etc. is added. The eluting solution may be a basic solution having pH of about 7 to about 13, for example, a basic solution of pyridine or containing pyridine as the main component, and an acidic solution having pH in the range of about 2 to about 7, for example, an acidic solution of pyridine-acetic acid, acetic acid, formic acid, hydrochloric acid and containing these as the main component.

The cation exchange resin to be employed may include, for example, carboxymethyl cellulose, carboxymethyl Sephadex (trade name, produced by Pharmacia P-L Biochemicals) and SP-Sephadex (trade name, produced by Pharmacia P—L Biochemicals).

The gel chromatography of the treatment (c) may preferably be conducted in a volatile buffer such as of acetic acid, ammonium hydrogen carbonate, ammonium formate, etc. Examples of the column are Biogel P 10 (trade name, produced by Bio-rad Co.) and Sephadex G 50 (trade name, produced by Pharmacia P—L Biochemicals).

The high performance liquid chromatography (HPLC) can be carried out in a conventional manner, and a linear concentration gradient elution effected with the use of a solvent such as ammonium formate (10 mM—1.0 M) — acetonitrile, water — acetonitrile — 10% trifluoroacetic acid (TFA) (90:10:1—40:60:1), etc. to recover the fractions having calcitonin-like physiological activity.

Assay of each fraction in the respective purification steps as described above was conducted by use of the radioimmunoassay, utilizing the cross-reactivity of the anti-eel CT serum with the CT of this invention, optionally combined with biological activity assay.

In radioimmunoassay, by use of the eel CT which is an antigen, rabbits are immunized for several times and exsanguinated after immunization to obtain an antiserum. The radioimmunoassay as herein mentioned refers to the method in which the quantity of the CT of this invention is detected through the competitive reactions between the CT of this invention and the radio-labelled antigen with an antibody (antiserum) of the eel CT. On the other hand, the labelled antigen can be prepared according to the Chloramine T method in which radioactive iodine ($^{125}I$) is bound to the tyrosine moiety of the eel CT. Next, several kinds of solutions of the CT extract or respective fractions from the respective steps (test sample), which are suitably diluted, are allowed to react well (4°C, overnight) with the anti-eel CT serum obtained above, and the quantity of the $^{125}I$-eel CT not bound to the antibody contained in this supernatant is measured by a γ-counter, whereby the relative CT activity contained in the test sample can be determined. According to the radioimmunoassay, the CT quantity consumed by assay can not only be lowered by about 1/100 as compared with the biological activity assay, but also it has the advantages of simple manual operation, excellent quantitation and reproducibility and short time required for assay. Thus, the radioimmunoassay enables easy and rapid separation and purification of the novel CT in this invention.

This invention is described in more detail by referring to the following Examples, by which this invention is not limited.

## Example 1

A) *Starting material*

Chickens (3384 in number) for meat of 10 weeks-old were exsanguinated and immediately thereafter ultimobranchial glands were taken out. After fat was removed as far as possible, the product was freezed with dry ice. The ultimobranchial glands obtained amounted to about 179.11 g.

B) *Extraction step*

The ultimobranchial glands obtained according to the above method were subdivided into portions (each corresponding to 500 chickens'), and each portion was immersed in 1 N acetic acid [5-fold (by weight ratio) of the weight of ultimobranchial glands], homogenized by Polytron (trade name, produced by Kinematica GmbH, Switzerland) and then subjected to centrifugation (9,000 rpm, 30 minutes). Subsequently, the intermediate layer was collected so that no fat may be contained therein, and a part of the extract was lyophilized.

A part of the lyophilized powder was dissolved in a 0.1 N formic acid and its biological activity was measured to be 1.0 to 1.2 MRCU/one chicken.

C) *Purification step*

After acetone (1800 ml) was gradually added to the aqueous acetic acid solution (900 ml) containing the desired physiologically active substance as prepared above, the mixture was left to stand overnight to permit higher molecular compounds to precipitate. After centrifugation (9000 rpm, 30 minutes), the supernatant was decanted.

The supernatant was poured onto a column (diameter 3.2 cm × height 22 cm) of SP—Sephadex C—25 ($H^+$-form) preequilibrated with 1 N acetic acid. By this operation, acidic substances were removed and basic substances containing the desired physiologically active susbtance were absorbed ionically onto SP—Sephadex C—25. Then, the adsorbed substances were eluted with 2 M pyridine. The eluate obtained was found to have a relatively lower CT activity. Next, elution was effected with 2 M pyridine-acetic acid (pH 5.0), and the eluate obtained was found to have a relatively higher CT activity.

In the experiments conducted hereinafter, the aforesaid latter eluate was employed. The eluate was concentrated, added with 1 N acetic acid and lyophilized. A solution of 1370 mg of the freeze-dried powder dissolved in 15 ml of 1 N acetic acid was poured onto a column (diameter 5.0 cm × height 130 cm) previously filled with Sephadex G—50 (Fine) and equilibrated with 1 N acetic acid. Elution was conducted under the conditions of an eluting solution of 1 N acetic acid and an eluting rate of 25 ml/h. The eluates were collected in fractions each of 15 ml/vial, and immunoractive fractions (fraction No. 50—57) were collected and lyophilized to obtain 20.8 mg of powder.

The CT quantity of the present invention immunologically reacted as calculated from the above assay was 692 µg (corresponding to 3384 chickens).

The powder (20.8 mg) was dissolved in 1.5 ml of a solution A (as hereinafter described) and this solution was subjected to HPLC following the measurement conditions described as follows:

Column: TSK GEL CM 2 SW produced by Toyo Soda Kogyo K.K. (4.6 mmϕ × 250 mm);
Flow rate: 2.0 ml/min.;
Differential pressure: 35 kg/cm$^2$;
Eluent:
Solution A (10 mM ammonium formate:acetonitrile = 9:1) and
Solution B (1.0 M ammonium formate: acetonitrile = 9:1) were used for carrying out the linear gradient elution from the Solution A to the Solution B one minute after injection of the sample.

In the result obtained, the activity based on the radioimmunoassay was identical with the absorbance derived from the peptide at 280 nm according to the adsorption method. Further, the sample was divided into three portions to repeat HPLC similarly under the following conditions.

Column: Chemcosorb 5 ODS—H produced by Chemco Co. (4.6 mmφ × 250 mm);

Eluate: 0.5 ml/vial;

Flow rate: 1.0 ml/min.;

Differential pressure: 140 kg/cm$^2$;

Eluent:

Solution A (water:acetonitrile:10% TFA = 90:10:1) and

Solution B (water:acetonitrile:10% TFA) were employed for carrying out the linear gradient elution 8 minutes after injection of the sample.

As the result, strong absorbances ($A_{280}$, $A_{210}$) based on the peptide were recognized in the active fraction (No. 40) [retention time 26 min. (after the gradient)]. The active fraction was lyophilized to obtain 106 μg of active powder. The biological activity of this powder was found to be 5300 MRCU/mg.

The analytical values of amino acids in the active powder as obtained above are shown in Table 1.

These were measured according to the method, in which 3.5 μg of the active powder was hydrolyzed with 6 N hydrochloric acid 110°C for 22 hours and, after removal of hydrochloric acid, the hydrolyzate was dissolved in 50 μl of 0.2 N citrate buffer (pH 3.25) and the whole amount was provided for analysis.

Further, in a conventional manner, according to the well-known protein determination method by carrying out primarily Edman degradation, the amino acid sequence was determined successively from the amino group end, simultaneously with determination of the carboxylic end by use of carboxy peptidase, to determine the amino acid sequence of the novel calcitonin represented by the above formula (I).

TABLE 1

Amino acid composition of the calcitonin of the invention

| Amino acid | Molar ratio* | Taken value |
|---|---|---|
| Asp | 1.08 | 1 |
| Thr | 3.73 | 4 |
| Ser | 2.80 | 3 |
| Glu | 2.93 | 3 |
| Pro | 1.89 | 2 |
| Gly | 3.03 | 3 |
| Ala | 2.00 | 2 |
| Val | 1.95 | 2 |
| Met | 0.03 | 0 |
| Ile | 0.04 | 0 |
| Leu | 4.91 | 5 |
| Tyr* | 1.00 | 1 |
| Lys | 1.90 | 2 |
| His | 1.01 | 1 |
| Arg | 1.03 | 1 |
| Cys** | 1.80 | 2 |

*) Determined by the calculation on the basis of tyrosine (Tyr) value as 1.00;
**) Method for measurement of Cys·amino acid analysis after performic acid oxidation [s. Moore, J. Biol. Chem., *238*, 235 (1963)].

Example 2

Similarly as in Example 1, 625 ml of an extracted acetic acid solution containing the CT of this invention was obtained from chicken ultimobranchial glands (500 chickens).

This solution was injected into a SP—Sephadex C—25 column (diameter 2.0 cm × height 17 cm) previously equilibrated with 1 N acetic acid. Then, the column was subjected to elution with 2 M pyridine, followed further by elution with 2 M pyridine-acetic acid (pH 5.0). The CT of this invention was found to be contained in the latter eluate.

To 120 ml of the eluate was added 240.ml of acetone, and the mixture after being left to stand overnight was centrifuted (9000 rpm, 30 minutes). The supernatant was collected and lyophilized to obtain 200 mg of powder.

The lyophilized powder was dissolved in 1 N acetic acid to prepare a solution of about 100 mg/ml, and injected into a column diameter 1.2 cm × height 103 cm) of Sephadex G—50 (Fine). The elution conditions employed were a 1 N acetic acid for the eluent and 5.7 ml/h for the eluting rate. The active fractions as detected by radioimmunoassay were recovered and lyophilized to obtain 3.0 mg of lyophilized powder. The CT quantity of this invention immunologically reacted as calculated from the above assay was found to be 120 mg. Final purification of this powder was performed by reverse phase HPLC on a column of Chemcosorb 5 ODS—H (4.6 mmϕ × 250 mm, trade name, produced by Chemco Co.), according to the same method as in Example 1 to obtain 24 µg of active powder of CT. This powder had a biological activity of 4800 MRCU/mg.

Further, amino acid analysis was conducted and amino acid sequence was also determined to confirm that this active powder is the CT of this invention.

Example 3

Similarly as in Example 1, 625 ml of extracted acetic acid containing the CT of this invention was obtained from chicken ultimobranchial glands (500 g in number) and lyophilized to obtain 1.5 g of powder.

The powder was dissolved in 15 ml of 1 N acetic acid and then subjected to centrifugation (3000 rpm, 20 minutes). The supernatant was injected into a column (diameter 1.2 cm × height 103 cm) of Sephadex G—50 (Fine).

The active fraction was adsorbed on a column (3.2 mmϕ × 22 cm) of SP—Sephadex C—25 ($H^+$-form) and 80 μg of powder having CT activity was obtained similarly as in Example 1.

For this powder, the high performance liquid chromatography operation was practiced according to the same procedure as in Example 1 to obtain 13-μg of active powder of CT. This powder was found to have a biological activity of 5100 MRCU/mg.

Further, amino acid analysis was conducted and also amino acid sequence was determined to confirm that this active powder is the CT of this invention.

**Claims**

1. A novel calcitonin represented by the following formula (I) and its salt:

H-Cys-Ala-Ser-Leu-Ser-Thr-Cys-Val-Leu-Gly-Lys-Leu-Ser-Gln-Glu-Leu-His-
Lys-Leu-Gln-Thr-Tyr-Pro-Arg-Thr-Asp-Val-Gly-Ala-Gly-Thr-Pro-$NH_2$ (I)

wherein Cys represents cysteine, Ala alanine, Ser serine, Leu leucine, Thr threonine, Val valine, Gly glycine, Lys lysine, Gln glutamine, Glu glutamic acid, His histidine, Tyr tyrosine, Pro proline, Arg arginine and Asp aspartic acid.

2. A process for collecting a novel calcitonin represented by the following formula (I) and its salt:

H-Cys-Ala-Ser-Leu-Ser-Thr-Cys-Val-Leu-Gly-Lys-Leu-Ser-Gln-Glu-Leu-His-
Lys-Leu-Gln-Thr-Tyr-Pro-Arg-Thr-Asp-Val-Gly-Ala-Gly-Thr-Pro-$NH_2$ (I)

wherein Cys represents cysteine, Ala alanine, Ser serine, Leu leucine, Thr threonine, Val valine, Gly Glycine, Lys lysine, Gln glutamine, Glu glutamic acid, His histidine, Tyr tyrosine, Pro proline, Arg arginine and Asp aspartic acid,
which comprises extracting an avian ultimobranchial gland with an aqueous acidic solution and thereafter applying at least one of the following treatments (a) to (d) on the extract:

(a) to precipitate and remove higher molecular weight compounds by addition of a hydrophilic organic solvent to the aqueous solution containing the desired product;

(b) to recover basic fractions with the use of a cation exchange resin;

(c) to recover fractions with molecular weights of 3000 to 4000 by gel chromatography; and

(d) to recover fractions having calcitonin-like physiological activity by high performance liquid chromatography.

**Patentansprüche**

1. Eine neues Calcitonin der Formel (I) und dessen salz

H-Cys-Ala-Ser-Leu-Ser-Thr-Cys-Val-Leu-Gly-Lys-Leu-Ser-Gln-Glu-Leu-His-
Lys-Leu-Gln-Thr-Tyr-Pro-Arg-Thr-Asp-Val-Gly-Ala-Gly-Thr-Pro-$NH_2$ (I)

worin Cys Cystein, Ala Alanin, Ser Serin, Leu Leucin, Thr Threonin, Val Valin, Gly Glycin, Lys Lysin, Gln Glutamin, Glu Glutaminsäure, His Histidin, Tyr Tyrosin, pro prolin, Arg Arginin und Asp Asparaginsäure bedeuten.

2. Verfahren zum Sammeln eines neuen Calcitonins der allgemeinen Formel (I) und von Salzen davon:

H-Cys-Ala-Ser-Leu-Ser-Thr-Cys-Val-Leu-Gly-Lys-Leu-Ser-Gln-Glu-Leu-His-
Lys-Leu-Gln-Thr-Tyr-Pro-Arg-Thr-Asp-Val-Gly-Ala-Gly-Thr-Pro-$NH_2$ (I)

worin Cys Cystein, Ala Alanin, Ser Serin, Leu Leucin, Thr Threonin, Val Valin, Gly Glycin, Lys Lysin, Gln

Glutamin, Glu Glutaminsäure, His Histidin, Tyr Tyrosin, pro prolin, Arg Arginin und Asp Asparaginsäure bedeuten, umfassend das Extrahieren von ultimobranchialen Drüsen von Vögeln mit einer wässrigen sauren Lösung und anschliessend Durchführung wenigstens einer der nachfolgenden Behandlungen (a) bis (d) an dem Extrakt:

(a) zum Ausfällen und Entfernen von höhermolekulargewichtigen Verbindungen, die Zugabe eines hydrophilen organischen Lösungsmittels zu der wässrigen Lösung, enthaltend das gewünschte Produkt;

(b) zur Gewinnung von basischen Fraktionen die Verwendung eine Kationenaustauscherharzes;

(c) zur Gewinnung von Fraktionen mit Molekulargewichten von 3.000 bis 4.000, die Anwendung von Gelchromatografie; und

(d) zur Gewinnung von Fraktionen mit einer calcitoninähnlichen physiologischen Aktivität, die Durchführung einer Hochleistungs-Flüssigchromatografie.

**Revendications**

1. Nouvelle calcitonine représentée par la formule (I) suivante, et son sel:

H-Cys-Ala-Ser-Leu-Ser-Thr-Cys-Val-Leu-Gly-Lys-Leu-Ser-Gln-Glu-Leu-His-
Lys-Leu-Gln-Thr-Tyr-Pro-Arg-Thr-Asp-Val-Gly-Ala-Gly-Thr-Pro-$NH_2$ (I)

où Cys représente la cystéine, Ala l'alanine, Ser la sérine, Leu la leucine, Thr la thréonine, Val la valine, Gly la glycine, Lys la lysine, Gln la glutamine, Glu l'acide glutamique, His l'histidine, Tyr la tyrosine, Pro la proline, Arg l'arginine et Asp l'acide aspartique.

2. Procédé d'isolement d'une nouvelle calcitonine, représentée par la formule (I) suivante, et de son sel:

H-Cys-Ala-Ser-Leu-Ser-Thr-Cys-Val-Leu-Gly-Lys-Leu-Ser-Gln-Glu-Leu-His-
Lys-Leu-Gln-Thr-Tyr-Pro-Arg-Thr-Asp-Val-Gly-Ala-Gly-Thr-Pro-$NH_2$ (I)

où Cys représente la cystéine, Ala l'alanine, Ser la sérine, Leu la leucine, Thr la thréonine, Val la valine, Gly la glycine, Lys la lysine, Gln la glutamine, Glu l'acide glutamique, His l'histidine, Tyr la tyrosine, Pro la proline, Arg l'arginine et Asp l'acide aspartique,

qui comporte son extraction hors d'une glande ultimobranchiale aviaire à l'aide d'une solution aqueuse acide, et l'application ultérieure à l'extrait d'au moins un des traitements suivants (a) à (d):

(a) précipitation et élimination des composés de masse moléculaire élevée, par addition d'un solvant organique hydrophile à la solution aqueous contenant le product désiré;

(b) récupération des fractions basiques par utilisation d'une résine échangeuse de cations;

(c) récupération des fractions présentant des masses moléculaires de 3000 à 4000 par chromatogrpahie sur gel; et

(d) récupération des fractions présentant une activité physiologique analogue à celle de la calcitonine par chromatographie liquide de haute performance.